(19) Europäisches Patentamt
European Patent Office
Office européen des brevets

(11) Veröffentlichungsnummer: **0 174 274**
**B1**

(12) # EUROPÄISCHE PATENTSCHRIFT

(45) Veröffentlichungstag der Patentschrift: **23.11.88**

(51) Int. Cl.⁴: **C 07 C 127/22**, C 07 C 93/14, A 01 N 47/34

(21) Anmeldenummer: **85810382.3**

(22) Anmeldetag: **26.08.85**

(54) **Phenylbenzoylharnstoffe.**

(30) Priorität: **31.08.84 CH 4179/84**

(43) Veröffentlichungstag der Anmeldung: **12.03.86 Patentblatt 86/11**

(45) Bekanntmachung des Hinweises auf die Patenterteilung: **23.11.88 Patentblatt 88/47**

(84) Benannte Vertragsstaaten: **AT BE CH DE FR GB IT LI NL**

(56) Entgegenhaltungen:
**EP-A-0 003 099**
**EP-A-0 023 884**
**EP-A-0 071 279**
**EP-A-0 093 976**
**DE-A-2 726 684**

**Die Akte enthält technische Angaben, die nach dem Eingang der Anmeldung eingereicht wurden und die nicht in dieser Patentschrift enthalten sind.**

(73) Patentinhaber: **CIBA- GEIGY AG, Klybeckstrasse 141, CH- 4002 Basel (CH)**

(72) Erfinder: **Ehrenfreund, Josef, Dr., Amselstrasse 11, CH- 4123 Allschwil (CH)**

**EP 0 174 274 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue N-[3-Halogen-4-(hexafluorpropyloxy)-5-trifluormethyl]-phenyl-N'-benzoylharnstoffe, Verfahren zu ihrer Herstellung und ihre Verwendung in der Schädlingsbekämpfung.

Diese erfindungsgemässen Verbindungen haben die Formel I

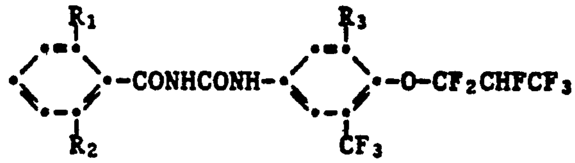 (I),

worin
$R_1$ Wasserstoff oder Halogen und
$R_2$ und $R_3$ Halogen bedeuten.

Unter Halogen sind im Rahmen der vorliegenden Erfindung Fluor, Chlor und Brom zu verstehen, insbesondere Fluor und Chlor für die Substituenten $R_1$, und $R_2$ und Chlor und Brom für den Substituenten $R_3$.

Wegen ihrer Wirkung als Schädlingsbekämpfungsmittel bevorzugt sind Verbindungen der Formel I, die dadurch gekennzeichnet sind, dass
$R_1$ Wasserstoff, Fluor oder Chlor,
$R_2$ Fluor oder Chlor und
$R_3$ Chlor bedeuten.

Besondere Erwähnung verdienen auch diejenigen Verbindungen der Formel I, bei denen
$R_1$ für Wasserstoff, Fluor oder Chlor,
$R_2$ für Fluor oder Chlor und
$R_3$ für Brom stehen.

Die Verbindungen der Formel I können nach an sich bekannten Verfahren hergestellt werden (vgl. u. a. die DE-A-2 123 236, DE-A-2 601 780 und EP-A-13 414).

So kann man z.B. eine Verbindung der Formel I erhalten durch Umsetzung
a) einer Verbindung der Formel II

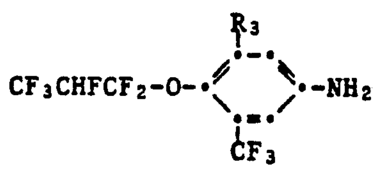 (II)

mit einer Verbindung der Formel III

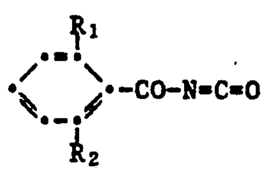 (III),

b) einer Verbindung der Formel IV

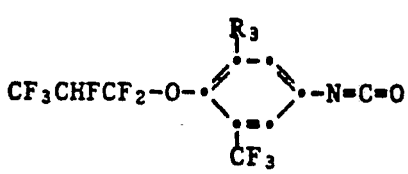 (IV)

mit einer Verbindung der Formel V

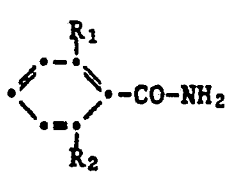 (V)

oder

c) einer Verbindung der Formel II mit einer Verbindung der Formel VI

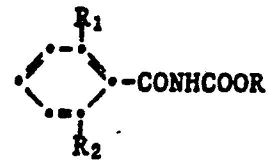 (VI).

In den obigen Formeln II, III, IV, V und VI haben die Reste $R_1$, $R_2$ und $R_3$ die unter Formel I vorstehend angegebenen Bedeutungen und R bedeutet einen gegebenenfalls mit Halogen, vorzugsweise Chlor, substituierten niederen oder mittleren $C_1$-$C_8$-Alkylrest.

Die erwähnten Verfahren a) bis c) können vorzugsweise unter normalen Druck und in Gegenwart eines organischen Lösungs- oder Verdünnungsmittels durchgeführt werden. Als Lösungs- oder Verdünnungsmittel eignen sich z. B. Äther und ätherartige Verbindungen, wie Diäthyläther, Dipropyläther- Dibutyläther- Dioxan, Dimethoxyäthan und Tetrahydrofuran; N,N-dialkylierte Carbonsäureamide; aliphatische, aromatische sowie halogenierte Kohlenwasserstoffe- insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z. B. Aceton, Methyläthylketon, Methylisopropylketon und Methylisobutylketon. Verfahren a) wird im allgemeinen bei einer Temperatur von - 10 bis 100°C, vorzugsweise zwischen 15 und 25°C, gegebenenfalls in Gegenwart einer organischen Base, z. B. Triäthylamin, durchgeführt. Die Durchführung von Verfahren b) erfolgt bei einer Temperatur von 0 bis 150°C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium. Für die Umsetzung der Urethane VI gemäss Verfahren c) wählt man in allgemeinen Temperaturen zwischen 60°C und dem Siedepunkt des Reaktionsgemisches. Als Lösungsmittel eignen sich vor allem aromatische Kohlenwasserstoffe, wie Toluol, Xylol, Chlorbenzol usw.

Die Ausgangsstoffe der vorstehenden Formeln II, III, IV, V und VI sind bekannt oder lassen sich, falls sie neu sind, analog bekannten Verfahren herstellen. Bei den substituierten Anilinen der Formel II handelt es sich um neue Verbindungen, die ebenfalls einen Gegenstand der vorliegenden Erfindung bilden und gemäss literaturbekannten Verfahren herstellbar sind, indem man z. B. 2-Halogen-6-trifluormethyl-4-nitrophenol [analog J. Org. Chem. 27 (1962), 4660] in Anwesenheit von Acetanhydrid hydriert und das entstandene 2-Halogen-6-trifluormethyl-4-acetaminophenol mit Hexafluorpropylen analog der in J. Am. Chem. Soc. 73 (1951), 5831, beschriebenen Arbeitsweise veräthert. Anschliessend wird die N-Acetylgruppe in üblicher Weise abgespalten, um zu dem Anilin der Formel II zu gelangen.

Es wurde gefunden, dass die erfindungsgemässen Verbindungen der Formel I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Schädlingen.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen der Formel I entspricht einer Abtötungsrate (Mortalität) von mindestens 50 - 60 % der erwähnten Schädlinge.

Neben ihrer sehr günstigen Wirkung gegenüber Fliegen, wie z. B. Musca domestica, und Mückenlarven eignen sich Verbindungen der Formel I vor allem zur Bekämpfung von pflanzenschädigenden Frassinsekten, in Zier- und Nutzpflanzungen, insbesondere in Baumwollkulturen (z. B. Spodoptera littoralis und Heliothis virescens) sowie in Gemüsekulturen (z. B. Leptinotarsa decemlineata und Pieris brassicae).

Hervorzuheben ist besonders die larvizide und ovizide Wirkung von Verbindungen der Formel I. Werden Verbindungen der Formel I von adulten Insekten mit dem Futter aufgenommen, so ist in vielen Fällen, insbesondere bei Coleopteren, wie z. B. Anthonomus grandis, eine verminderte Ei-Ablage und/oder reduzierte Schlupfrate festzustellen.

Die Verbindungen der Formel I eignen sich weiterhin zur Bekämpfung von Ektoparasiten, z. B. Lucilia sericata, sowie von Zecken an Haus- und Nutztieren, z. B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z. B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern,

Stäubemitteln, Granulaten, auch Verkapselungen in z. B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d. h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Äther und Ester, wie Ähanol, Äthylenglykol, Äthylenglykolmonomethyl- oder äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z. B. für Stäubemittel und dispergierbare Pulver, werden in der Regel natürliche Gesteinsmehle vervendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$ - $C_{22}$), wie z. B. die Na- oder K-Salze der Öl- oder Stearinsäure, oder von natürlichen Fettsäuregemischen, die z. B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Äthylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z. B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z. B. Salze des Phosphorsäureesters eines p-Nonylphenol-(4-4)-Äthylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Rohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Äthylenglykoläthergruppen und 10 bis 100 Propylenglykoläthergruppen enthaltenden Polyäthylenoxid-Addukte an Polypropylenglykol, Äthylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Äthylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z. B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

**Beispiel 1:**

**a) Herstellung der Ausgangsverbindung 3-Chlor-4-(1,1,2,3,3,3-hexafluorpropyloxy)-5-(trifluormethyl)-anilin:**

Es werden 42,2 g 3-Chlor-4-(1,1,2,3,3,3-hexafluorpropyloxy)-5-(trifluormethyl)-acetaminobenzol in einer Mischung aus 200 ml konzentrierter Salzsäure und 400 ml Äthanol während 16 Stunden am Rückfluss gekocht. Anschliessend wird die Hauptmenge des Lösungsmittels abdestilliert, mit 10 %-iger Natronlauge alkalisiert und mit Äther extrahiert. Nach Trocknen über Natriumsulfat und Verdampfen des Lösungsmittels im Vakuum wird der Rückstand fraktioniert destilliert. Man erhält die Titelverbindung als gelbliches Öl mit einem Siedepunkt von 115 - 120° C / $4 \cdot 10^2$ Torr (eine Verbindung der Formel II).

**b) Herstellung von N-[3-Chlor-4-(hexafluorpropyloxy)-5-trifluormethyl-phenyl]-N'-2,6-difluorbenzoyl-harnstoff:**

Es werden 3,6 g des nach a) erhaltenen 3-Chlor-4-(hexafluorpropyloxy)-5-(trifluormethyl)-anilins in 10 ml trockenen Diäthyläther vorgelegt; unter Rühren werden bei Raumtemperatur 1,82 g 2,6-Difluorbenzoylisocyanat zugetropft. Nach 3 Stunden wird der gebildete kristalline Niederschlag abfiltriert und mit wenig Äther ausgewaschen. Man erhält so die Titelverbindung der Formel

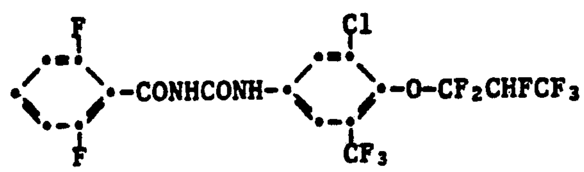

als weisse Kristalle mit einem Schmelzpunkt von 162 - 163° C (Verbindung Nr. 1).

Entsprechend der vorstehend beschriebenen Arbeitsweisen werden auch die folgenden Verbindungen der Formel I hergestellt:

| Verbindung Nr. | | Smp. [° C] |
|---|---|---|
| 2 | 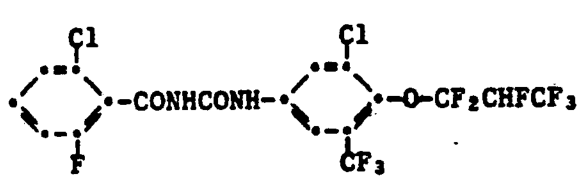 | 166-168 |
| 3 | 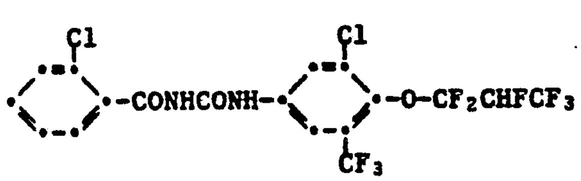 | 149,5-151 |

4 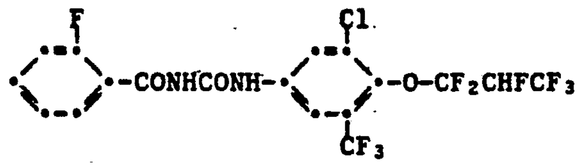 118-120

5 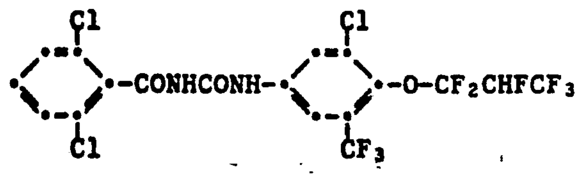 191,5-193

**Beispiel 2:**

**a) Herstellung der Ausgangsverbindung 3-Brom-4-(1,1,2,3,3,3-hexafluorpropyloxy)-5-(trifluormethyl)-anilin:**

Es werden 22,7 g 3-Brom-4-(1,1,2,3,3,3-hexafluorpropyloxy)-5-(trifluormethyl)-acetaminobenzol in einer Mischung aus 100 ml konzentrierter Salzsäure und 300 ml Äthanol während 4 Stunden am Rückfluss erhitzt. Anschliessend wird die Hauptmenge des Lösungsmittels abdestilliert, mit 10 %-iger Natronlauge alkalisiert und mit Äther extrahiert. Nach Trocknen über Natriumsulfat und Verdampfen des Lösungsmittels im Vakuum wird der Rückstand fraktioniert destilliert. Man erhält die Titelverbindung als gelbliches Öl mit einem Siedepunkt von 115 -120°C / $5 \cdot 10^{-2}$ Torr (eine Verbindung der Formel II).

**b) Herstellung von N-[3-Brom-4-(hexafluorpropyloxy)-5-trifluormethyl-phenyl]-N'-2,6-difluorbenzoyl-harnstoff:** >e

Es werden 3,5 g des nach a) erhaltenen 3-Brom-4-(hexafluorpropyloxy)-5-(trifluormethyl)-anilins in 10 ml trockenem Diäthyläther vorgelegt; unter Rühren werden bei Raumtemperatur 1,52 g 2,6-Difluorbenzoylisocyanat zugetropft. Nach 3 Stunden wird der gebildete kristalline Niederschlag abfiltriert und mit wenig Äther ausgewaschen. Man erhält so die Titelverbindung der Formel

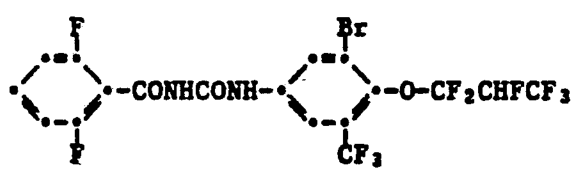

als weisse Kristalle mit einem Schmelzpunkt von 145 - 147°C (Verbindung Nr. 6).

Entsprechend der vor stehend beschriebenen Arbeitsweisen werden auch die folgenden Verbindungen der Formel I hergestellt:

| Verbindung Nr. | | Smp. [°C] |
|---|---|---|
| 7 | 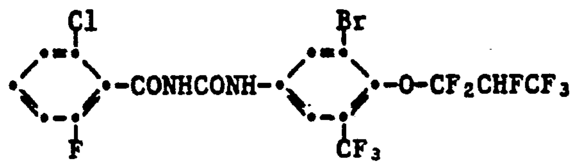 | 183,5-185 |
| 8 | 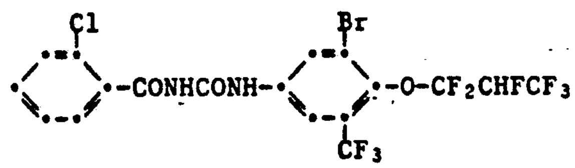 | 141-142 |
| 9 | 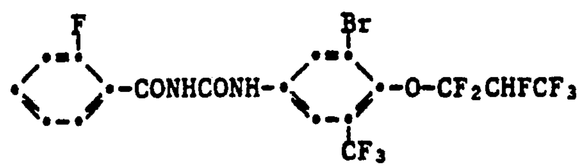 | |
| 10 | 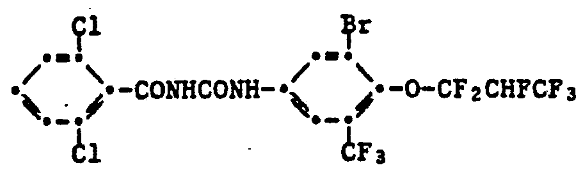 | |

**Beispiel 3:**

**Formulierungen für Wirkstoffe der Formel I gemäss den Beispielen 1 und 2 resp. Kombinationen dieser Wirkstoffe mit andern Insektiziden oder Akariziden (% = Gewichtsprozent):**

| 1. Spritzpulver | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläter (7 - 8 Mol AeO) | - | 2% | - |
| Hochdisperse Kieselsäure | 5 % | 10 % | 10 % |
| Kaolin | 62 % | 27 % | - |

Der Wirkstoff oder die Werkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2. Emulsions-Konzentrat

| | |
|---|---|
| Wirkstoff oder Wirkstoffkombination | 10 % |
| Octylphenolpolyäthylenglykoläther (4 - 5 Mol AeO) | 3 % |
| Ca-Dodecylbenzolsulfonat | 3 % |
| Ricinusölpolyglykoläther (36 Mol AeO) | 4% |
| Cyclohexanon | 30 % |
| Xylolgemisch | 50% |

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

| | |
|---|---|
| Wirkstoff oder Wirkstoffkombination | 10 % |
| Na-Ligninsulfonat | 2 % |
| Carboxymethylcellulose | 1% |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

| | |
|---|---|
| Wirkstoff odar Wirkstoffkombination | 40 % |
| Äthylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %-ige wässrige Formaldehyd-Lösung | 0,2 % |
| Silikonöl in Form einer 75 %-igen wässtigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Beispiel 4:**

**Wirkung gegen Musca domestica**

Je 50 g frisch zubereitetes Nährsubstrat für Maden werden in Becher eingewogen. Von einer 1 Gew.-%-igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff je 25 eintägige Maden von Musca domestica in die dss so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss den Beispielen 1 und 2 zeigen gute Wirkung im obigen Test.

**Beispiel 5:**

**Wirkung gegen Lucilia sericata**

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss den Beispielen 1 und 2 zeigen in diesem Test gute Wirkung gegen Lucilia sericata.

**Beispiel 6:**

**Wirkung gegen Aëdes aegypti**

Auf die Oberfläche von 150 ml Wasser, das sich in einem Behälter befindet, wird so viel einer 0,1 %-igen acetonischen Lösung des Wirkstoffes pipettiert, dass eine Konzentration von 400 ppm erhalten wird. Nach Verdunsten des Acetons wird der Behälter mit 30 bis 40 2-tägigen Aëdes-Larven beschickt. Nach 2 und 7 Tagen wird die Mortalität geprüft.

Verbindungen der Formel I gemäss den Beispielen 1 und 2 zeigen gute Wirkung im obigen Test.

**Beispiel 7:**

**Insektizide Frassgift-Wirkung**

Baumwollpflanzen (ca. 20 cm hoch) werden mit wässrigen Wirkstoffemulsionen (erhalten aus einem 10 %-igen emulgierbaren Konzentrat) besprüht, wobei die Wirkstoffemulsionen 0,75 bis 400 ppm der jeweils zu prüfenden Verbindung enthalten.

Nach dem Antrocknen des Belages werden die Baumwollpflanzen mit Spodoptera littoralis- und Heliothis virescens-Larven im dritten larvalen Stadium besetzt. Der Versuch wird bei 24°C und 60 % relativer Luftfeuchtigkeit durchgeführt. In Abständen von jeweils 24 Stunden werden Mortalität sowie Entwicklungs- und Häutungsstörungen der angesetzten Larven bestimmt.

Gegen Spodoptera zeigen eine 80 - 100 %-ige Wirkung in diesem Test die Verbindungen Nr. 1, 2 und 4 bei 0,75 ppm und die Verbindungen Nr. 3, 6, 7 und 8 bei 3,0 ppm. Gegen Heliothis zeigen eine 80 - 100 %-ige Wirkung die Verbindungen Nr. 1 und 6 bei 3,0 ppm, die Verbindungen Nr. 2 und 7 bei 12,5 ppm, die Verbindungen Nr. 3 und 8 bei 50 ppm und die Verbindung Nr. 4 bei 400 ppm.

**Beispiel 8:**

**Wirkung auf Spodoptera littoralis und Heliothis virescens (Larven und Eier)**

Es werden drei in Töpfen gezogene Baumwollpflanzen von ca. 15 - 20 cm Höhe mit einer sprühfähigen flüssigen Zubereitung des zu prüfenden Wirkstoffes in einer Konzentration von 400 ppm behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Innern des abgedeckten Gefässes wird so reguliert, dass sich kein Kondenswasser bilden kann. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;

b) 20 Larven von Spodoptera littoralis oder Heliothis virescens des dritten larvalen Stadiums;

c) zwei Eispiegeln von Spodoptera littoralis oder Heliothis virescens (dazu werden je 2 Blätter einer der Baumwoll-Pflanzen in einem beidseitig mit Gaze verschlossenen Plexiglaszylinder eingeschlossen); zwei Eispiegel von Spodoptera oder ein Teil eines Baumwollblattes mit darauf abgelegten Eiern von Heliothis werden zu den eingeschlossenen Blättern gegeben.

Nach 4 und 5 Tagen erfolgt die Auswertung gegenüber unbehandelten Kontrollen unter Berücksichtigung folgender Kriterien:

a) Anzahl der noch lebenden Larven, b) larvale Entwicklungs- und Häutungsstörungen, c) Frass-schaden (Schabfrass und Lochfrass), d) Schlupfrate (Anzahl der aus den Eiern geschlüpften Larven).

Verbindungen der Formel I gemäss den Beispielen 1 und 2 zeigen gute Gesamt-Wirksamkeit in obigem Test.

**Beispiel 9:**

**Ovizide Wirkung auf Spodoptera littoralis**

Auf Filterpapier abgelegte Eier von Spodoptera littoralis werden aus dem Papier ausgeschnitten und in eine 0,05 Gew. %-ige Lösung des Wirkstoffes in einem Aceton-Wasser-Gemisch (1 : 1) getaucht. Die so behandelten Eiablagen werden dann aus diesem Gemisch herausgenommen und bei 28°C und 60 % relativer Feuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen der Formel I gemäss den Beispielen 1 und 2 zeigen gute Wirkung im obigen Test.

**Beispiel 10:**

**Ovizide Wirkung auf Epilachna varivestis**

Es werden 20 Gew.-% Wirkstoff, 70 Gew.-% Xylol und 10 Gew.-% einer Mischung aus einem Reaktionsprodukt eines Alkylphenols mit Äthylenoxid und Calcium-dodecylbenzolsulfonat miteinander vermischt. Aus diesem Konzentrat werden wässrige Emulsionen enthaltend 800 ppm Wirkstoff hergestellt.

Jeweils ca. 100 auf Blätter von Phaseolus vulgaris frisch abgelegte Eier von Epilachna varivestis (mexikanischer Bohnenkäfer) werden mit den oben beschriebenen wässrigen Emulsionen (Konzentration 800 ppm Wirkstoff) angefeuchtet und leicht getrocknet.

In einem gelüfteten Gefäss werden die behandelten Gelege so lange gehalten, bis die gleichzeitig angesetzten unbehandelten Kontrollen geschlüpft sind. Unter einem Binocular erfolgt Auswertung hinsichtlich der erzielten prozentualen Abtötung.

Verbindungen der Formel I gemäss den Beispielen 1 und 2 zeigen gute Wirkung in obigem Test.

**Beispiel 11:**

**Ovizide Wirkung auf Heliothis virescens und Leptinotarsa decemlineata**

Entsprechende Mengenanteile einer benetzbaren pulverförmigen Formulierung, enthaltend 25 Gew.-% des zu prüfenden Wirkstoffes, werden mit jeweils so viel Wasser vermischt, dass sich eine wässrige Emulsion mit einer Wirkstoffkonzentration von 800 ppm ergibt.

In die wirkstoffhaltigen Emulsionen werden eintägige Eigelege von Heliothis auf Cellophan® bzw. Eigelege von Leptinotarsa auf Kartoffelblättern während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgelegt.

Verbindungen der Formel I gemäss den Beispielen 1 und 2 zeigen gute Wirkung in obigem Test.

**Beispiel 12:**

**Ovizide Wirkung auf Laspeyresia pomonella (Eier)**

Abgelegte Eier von Laspeyresia pomonella, die nicht älter als 24 Stunden sind, werden auf Filterpapier für 1 Minute in eine acetonisch-wässrige Lösung enthaltend 400 ppm des zu prüfenden Wirkstoffes eingetaucht. Nach dem Antrocknen der Lösung werden die Eier in Petrischalen ausgelegt und bei einer Temperatur von 28° C belassen. Nach 6 Tagen wird der prozentuale Schlupf aus den behandelten Eiern bewertet.

Verbindungen der Formel I gemäss den Beispielen 1 und 2 zeigen gute Wirkung in obigem Test.

**Beispiel 13:**

**Wirkung gegen Anthonomus grandis (Adulte)**

Zwei eingetopfte Baumwollpflanzen im 6-Blattstadium werden mit wässrigen benetzungsfähigen Emulsions-Zubereitungen, enthaltend 12,5 ppm des zu prüfenden Wirkstoffes besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Öffnungen mit Gaze abgedeckt sind, werden dann über die behandelten, mit den Testtieren besiedelten Pflanzen gestülpt, um ein Abwandern der Käfer zu verhindern. Die behandelten Pflanzen werden bei 25° C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen Nr. 1, 2, 6 und 7 zeigen 80 - 100 %-ige Wirkung (Mortalität) im obigen Test.

**Beispiel 14:**

**Wirkung gegen pflanzenschädigende Akariden: Tetranychus urticae (OP-sensible) und Tetranychus cinnabarinus (OP-tolerant).**

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) oder Tetranchus cinnabarinus (OP-tol.) belegt. (Die Toleranz bezieht sich auf die Verträglichkeit gegenüber Diazinon).

Die so behandelten infestierten Pflanzen werden mit einer Versuchslösung enthaltend 400 ppm der zu prüfenden Verbindung bis zur Tropfnässe besprüht.

Nach 24 Stunden und wiederum nach 7 Tagen werden Imagines und Larven (alle beweglichen Stadien) unter dem Binokular auf lebende und tote Individuen ausgewertet.

Man verwendet pro Konzentration und pro Testspezies eine Pflanze. Während des Versuchsverlaufs stehen die Pflanzen in Gewächshauskabinen bei 25° C.

Verbindungen der Formel I gemäss den Beispielen 1 und 2 zeigen in diesem Versuch gute Wirkung gegen Tetranychus urticae und Tetranychus cinnabarinus.

**Beispiel 15:**

**Reproduktions-Beeinflussung von Anthonomus grandis**

Adulte Anthonomus grandis, die nach dem Schlupf nicht älter als 24 Stunden waren, werden in Gruppen zu jeweils 25 Käfern in Käfige mit Gitterwänden überführt. Die mit den Käfern besetzen Käfige werden sodann während 5 bis 10 Sekunden in eine acetonische Lösung, enthaltend 800 ppm des zu prüfenden Wirkstoffes, eingetaucht. Nachdem die Käfer wieder trocken sind, werden sie zur Kopulation und Eiablage in abgedeckte und Futter enthaltende Schalen eingesetzt. Abgelegte Eier werden zwei- bis dreimal wöchentlich mit fliessendem Wasser ausgeschwemnt, gezählt, durch zwei- bis dreistündiges Einlegen in ein wässriges Desinfektionsmittel desinfiziert und dann in Schalen, die eine geeignete Larvaldiät enthalten, deponiert. Nach 7 Tagen wird untersucht, ob sich aus den deponierten Eiern Larven entwickelt haben.

Zur Ermittlung der Dauer des die Reproduktion beeinflussenden Effektes der zu prüfenden Wirkstoffe wird die Eiablage der Käfer während eines Zeitraums von etwa 4 Wochen überprüft. Die Bonitierung erfolgt anhand der Verminderung der Anzahl abgelegter Eier und der daraus geschlüpften Larven im Vergleich zu unbehandelten Kontrollen.

Verbindungen der Formel I gemäss den Beispielen 1 und 2 zeigen eine gute reproduktionsreduzierende Wirkung im obigen Test.

**Patentansprüche**

1. Verbindung der Formel I

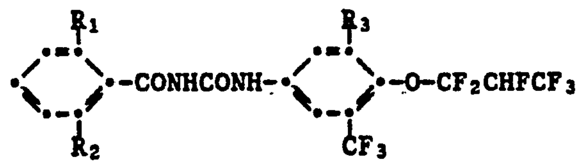

(I),

worin
$R_1$ Wasserstoff oder Halogen und
$R_2$ und $R_3$ Halogen
bedeuten.

2. Verbindung gemäss Anspruch 1, dadurch gekennzeichnet, dass
$R_1$ Wasserstoff, Fluor oder Chlor,
$R_2$ Fluor oder Chlor und
$R_3$ Chlor oder Brom
bedeuten.

3. Verbindung gemäss Anspruch 3, dadurch gekennzeichnet, dass
$R_1$ Wasserstoff, Fluor oder Chlor,
$R_2$ Fluor oder Chlor und
$R_3$ Chlor
bedeuten.

4. Verbindung gemäss Anspruch 3 der Formel

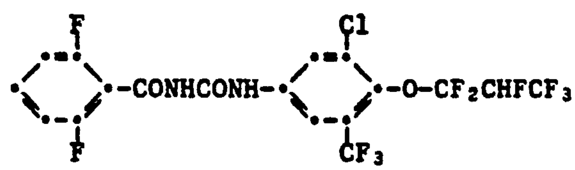

5. Verbindung gemäss Anspruch 3 der Formel

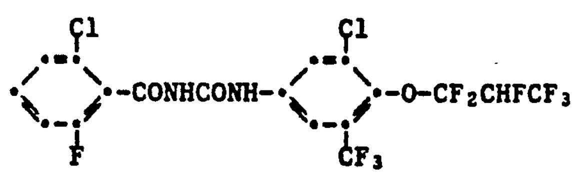

6. Verbindung gemäss Anspruch 2, dadurch gekennzeichnet, dass
$R_1$ Wasserstoff, Fluor oder Chlor,
$R_2$ Fluor oder Chlor und
$R_3$ Brom
bedeuten.

7. Verbindung gemäss Anspruch 6 der Formel

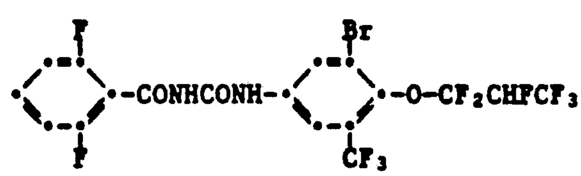

8. Verbindung gemäss Anspruch 6 der Formel

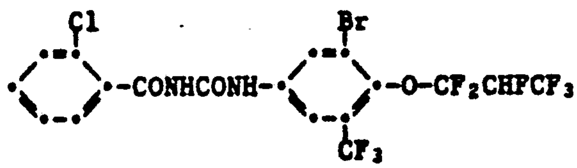

9. Verfahren zur Heretellung einer Verbindung der Formel I gemäss Anspruch 1, dadurch gekennzeichnet, dass man

a) eine Verbindung der Formel II

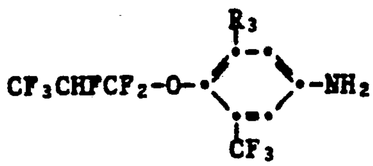

(II)

mit einer Verbindung der Formel III

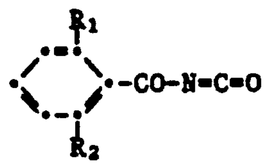

(III),

b) eine Verbindung der Formel IV

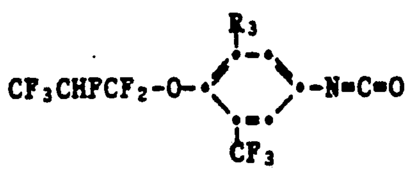

(IV)

mit einer Verbindung der Formel V

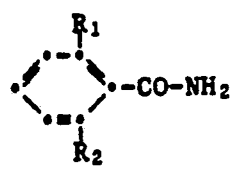

(V)

oder

c) eine Verbindung der Formel II mit einer Verbindung der Formel VI

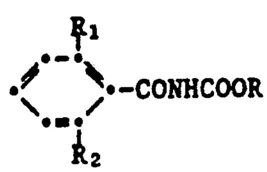

(VI).

umsetzt, wobei in den Formeln II, III, IV, V und VI die Reste $R_1$, $R_2$ und $R_3$ die unter Anspruch 1 angegebenen Bedeutungen haben und R für einen gegebenenfalls mit Halogen substituierten $C_1$ - $C_8$-Alkylrest steht.

10. Verbindung der Formel II

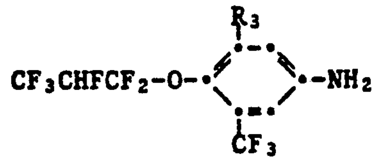

(II),

worin $R_3$ Halogen bedeutet.

11. Verbindung gemäss Anspruch 10, dadurch gekennzeichnet, dass $R_3$ Chlor oder Brom bedeutet.

12. Verbindung gemäss Anspruch 11 der Formel

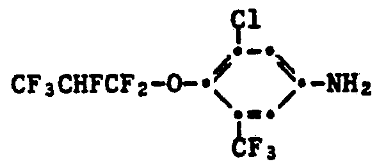

13. Verbindung gemäss Anspruch 11 der Formel

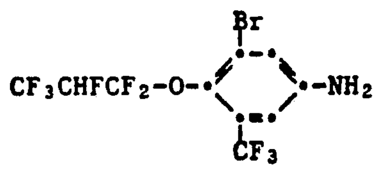

14. Schädlingsbekämpfungsmittel, welches als aktive Komponente eine Verbindung gemäss einem der Ansprüche 1 bis 8 zusammen mit geeigneten Trägern und/oder anderen Zuschlagstoffen enthält.

15. Verwendung einer Verbindung gemäss einem der Ansprüche 1 bis 8 zur Bekämpfung von Insekten und Vertretern der Ordnung Akarina an Tieren und Pflanzen.

16. Verwendung gemäss Anspruch 15 zur Bekämpfung von pflanzenschädigenden Insekten.

17. Verwendung gemäss Anspruch 16 zur Behämpfung von pflanzenschädigenden Insekten in Baumwoll- und Obstkulturen.

18. Verfahren zur Bekämpfung von Insekten und Vertretern der Ordnung Akarina, dadurch gekennzeichnet, dass man die Schädlinge bzw. deren verschiedene Entwicklungsstadien oder ihren Aufenthaltsort mit einer pestizid wirksamen Menge einer Verbindung der Formel I gemäss einem der Ansprüche 1 bis 8 oder mit einem Mittel enthaltend neben Zusatzund Trägerstoffen eine pestizid wirksame Menge dieser Verbindung, in Kontakt bringt oder behandelt.

**Claims**

1. A compound of formula I

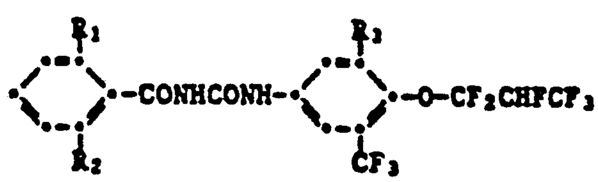

(I),

wherein $R_1$ is hydrogen or halogen and
$R_2$ and $R_3$ are halogen.

2. A compound according to claim 1, wherein
$R_1$ is hydrogen, fluorine or chlorine,
$R_2$ is fluorine or chlorine and
$R_3$ is chlorine or bromine.

3. A compound according to claim 2, wherein
$R_1$ is hydrogen, fluorine or chlorine,
$R_2$ is fluorine or chlorine, and
$R_3$ is chlorine.

4. A compound according to claim 3 of formula

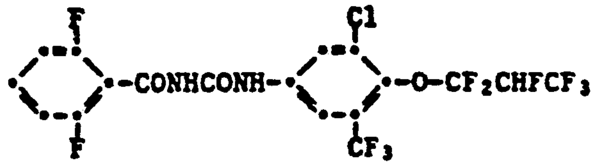

5. A compound according to claim 3 of formula

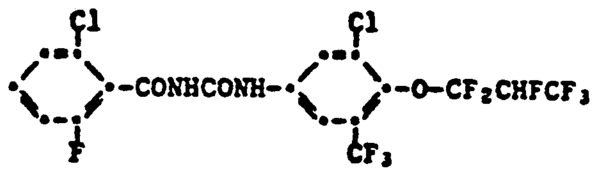

6. A compound according to claim 2, wherein
$R_1$ is hydrogen, fluorine or chlorine,
$R_2$ is fluorine or chlorine, and
$R_3$ is bromine.

7. A compound according to claim 6 of formula

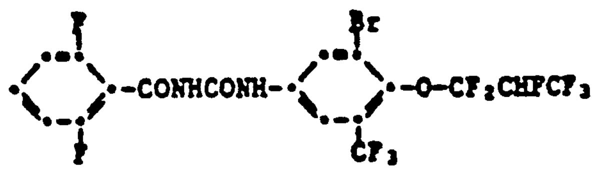

8. A compound according to claim 6 of formula

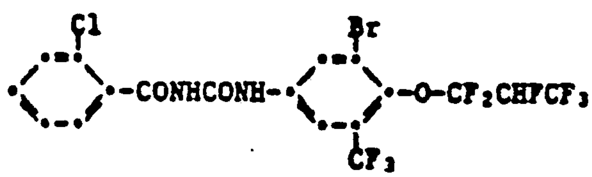

9. A process for the preparation of a compound of formula I according to claim 1, which comprises reacting
a) a compound of formula II

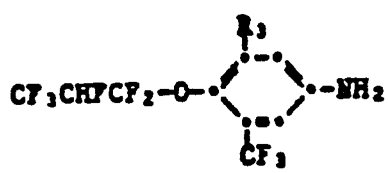

(II)

with a compound of formula III

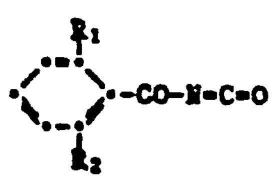

(III),

b) a compound of formula IV

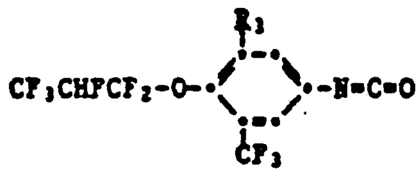

(IV)

with a compound of formula V

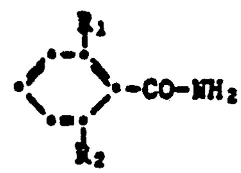

(V)

or

c) a compound of formula II with a compound of formula VI

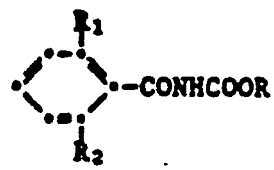

(VI)

in which formulae II, III, IV, V and VI the radicals $R_1$, $R_2$ and $R_3$ are as defined in claim 1 and R is a $C_1$ - $C_8$-alkyl radical which may be substituted by halogen.

10. A compound of formula II

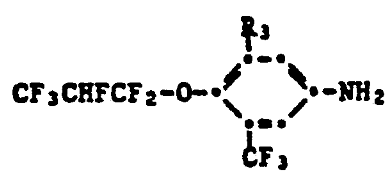

(II)

wherein $R_3$ is halogen.

11. A compound according to claim 10, wherein $R_3$ is chlorine or bromine.

12. A compound according to claim 11 of formula

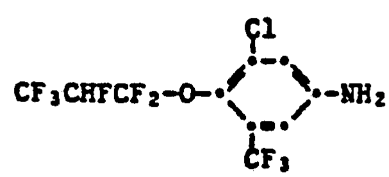

13. A compound according to claim 11 of formula

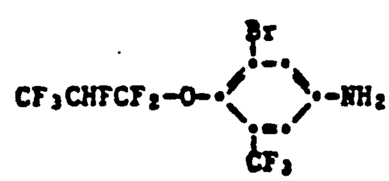

14. A pesticidal composition which contains as active component a compound according to any one of claims 1 to 8, together with suitable carriers and/or other adjuvants.

15. Use of a compound according to any one of claims 1 to 8 for controlling insects and representatives of the order Acarina on animals and plants.

16. Use according to claim 15 for controlling plant-destructive insects.

17. Use according to claim 16 for controlling plant-destructive insects in crops of cotton and fruit.

18. A method of controlling insects and representatives of the order Acarina, wherein the pests or their

various development stages or the locus thereof, are brought into contact with or treated with a pesticidally effective amount of a compound of formula I according to any one of claims 1 to 8, or with a composition which contains a pesticidally effective amount of such a compound, together with adjuvants and carriers.

**Revendications**

1. Composés de formule I

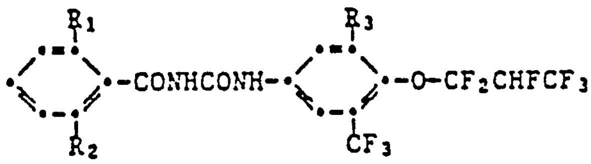

(I),

où

$R_1$ représente un hydrogène ou un halogène et
$R_2$ et $R_3$ représentent un halogène.

2. Composé selon la revendication 1, caractérisé en ce que
$R_1$ représente un hydrogène, fluor ou chlore,
$R_2$ représente un fluor ou un chlore et
$R_3$ représente un chlore ou un brome.

3. Composé selon la revendication 3, caractérisé en ce que
$R_1$ représente un hydrogène, un fluor ou un chlore,
$R_2$ représente un fluor ou un chlore et
$R_3$ représente un chlore.

4. Composé selon la revendication 3 de formule

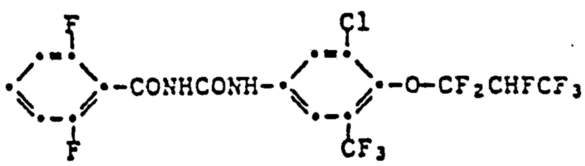

5. Composé selon la revendication 3 de formule

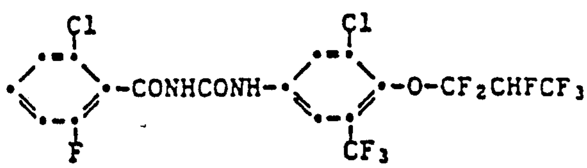

6. Composé selon la revendication 2, caractérisé en ce que
$R_1$ représente un hydrogène fluor ou chlore,
$R_2$ représente un fluor ou un chlore et
$R_3$ représente un brome.

7. Composé selon la revendication 6 de formule

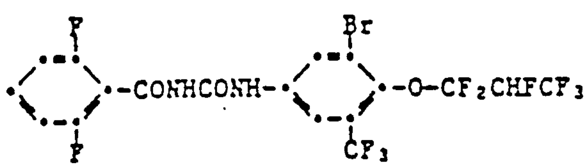

8. Composé selon la revendication 6 de formule

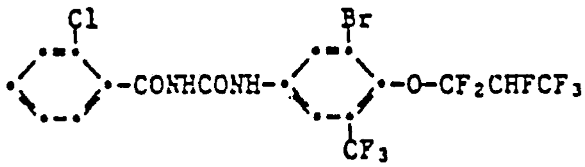

9. Procédé de préparation d'un composé de formule I selon la revendication 1, caractérisé en ce que

a) on fait réagir un composé de formule II

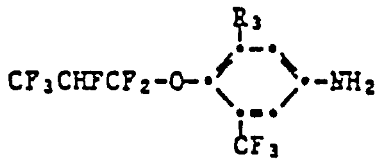 (II)

avec un composé de formule III

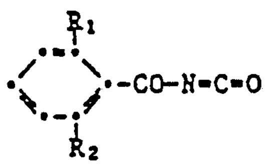 (III),

b) un composé de formule IV

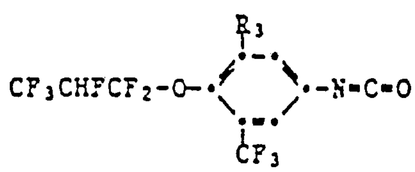 (IV)

avec un composé de formule V

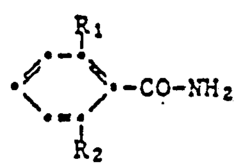 (V)

ou

c) un composé de formule II avec un composé de formule VI

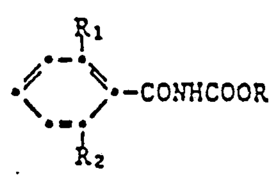 (VI).

où dans les formules II, III, IV, V et VI, les radicaux $R_1$ $R_2$ et $R_3$ ont les significations données pour la revendication 1 et R représente un radical alcoyle en $C_1$ à $C_8$ éventuellement substitué par un halogène.

10. Composé de formule II

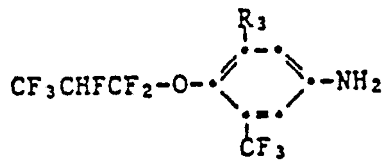 (II),

où $R_3$ représente un halogène.

11. Composé selon la revendication 10, caractérisé en ce que $R_3$ représente un chlore ou un brome.

12. Composé selon la revendication 11, de formule

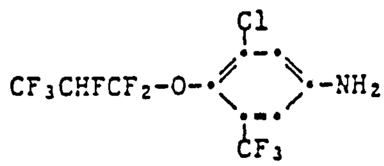

13. Composé selon la revendication 11 de formule

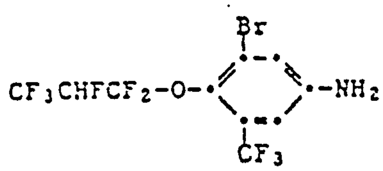

14. Agent de lutte anti-parasitaire, qui contient comme composant actif un composé selon l'une des revendications 1 à 8 avec des supports et/ou autres additifs appropriés.

15. Application d'un composé selon l'une des revendications 1 à 8 pour combattre les insectes et les représentants de l'ordre des acariens chez les animaux et les plantes.

16. Application selon la revendication 15 pour combattre les insectes parasites qui causent des dommages aux plantes.

17. Application selon la revendication 16 pour combattre les insectes parasites causant des dommages aux plantes dans les cultures de coton et de fruits.

18. Procédé de lutte contre les insectes et les représentants de l'ordre des acariens caractérisé en ce qu'on met en contact ou qu on traite les parasites ou leurs différents stades de développement ou leur habitat avec une quantité pesticide efficace d'un composé de formule I selon l'une des revendications 1 à 8 ou un agent contenant outre des additifs et des supports une quantité pesticide efficace de ce composé.